# EUROPEAN PATENT APPLICATION

(11) **EP 2 959 884 A1**
(43) Date of publication of application: **30.12.2015**
(21) Application number: 14173631.4
(22) Date of filing: 24.06.2014
(51) Int. Cl.: A61K 8/24, A61Q 11/00, A61K 8/19, A61K 8/21

(54) **New composition for remineralizing teeth**

(71) Applicant: Fialka, Pamela, 1180 Vienna (AT)
(72) Inventor: Fialka, Pamela, 1180 Vienna (AT)
(74) Representative: Loidl, Manuela Bettina

(57) **Abstract**

The present invention provides a composition comprising calcium carbonate, calcium fluoride and calcium phosphate for mineralizing teeth containing calcium carbonate in an amount of about 0.25-5g, calcium fluoride in an amount of about 0.1-3g and calcium phosphate in an amount of about 0.1-3g per unit.

## Description

The present invention provides a composition comprising calcium carbonate, calcium fluoride and calcium phosphate for mineralizing teeth containing calcium carbonate in an amount of about 0.25-5g, calcium fluoride in an amount of about 0.1-3g and calcium phosphate in an amount of about 0.1-3g per unit.

### BACKGROUND OF THE INVENTION

Caries lesions in dental enamel and demineralized dentin and cementum are common damages of the oral teeth which often lead to tissue loss.

Dental caries is a preventable and reversible infectious disease process, yet it continues to be the single most common chronic disease of childhood. Despite a decrease in caries prevalence and a decrease in untreated tooth decay in 6-19-year-olds in the United States, a 15.2% increase in disease was noted among the nation's youngest children aged 2-5 years.

Dental caries, not unlike periodontal diseases, is an infectious, transmissible, multifactorial disease of bacterial origin. Current evidence-based emphasis is on the need to recognize a carious lesion in its earliest stage before demineralization has produced a cavitated lesion that requires restoration by a dentist. As a result of current understanding of caries control, the dental hygienist's role as a prevention specialist is to determine the dental caries risk factors for patients of all ages and to introduce remineralization strategies into the patient's dental hygiene care plan. Conservative strategies of a concentrated program include initial infection control with a chlorhexidine rinse; extra daily fluoride exposures; placement of pit and fissure sealants where indicated; control of sucrose exposures; use of sugar substitutes, particularly xylitol-containing sugar-free chewing gum; and an emphasis on a daily bacterial plaque removal routine.

Dental erosion is defined as the loss of tooth substance by acid exposure not involving bacteria.

The acidic attack leads to an irreversible loss of dental hard tissue, which is accompanied by a progressive softening of the surface. The etiology of erosion is related to different behavioral, biological and chemical factors. Behavioral factors, such as special drinking habits, unhealthy lifestyle factors or occupational acid exposure, might modify the extent of dental erosion. Biological factors, such as saliva or acquired pellicle, act protectively against erosive demineralization. With regard to chemical factors, the modification of acidic solutions with ions, especially calcium, was shown to reduce the demineralization, but the efficacy depends on the other chemical factors, such as the type of acid. To enhance the remineralization of eroded surfaces and to prevent further progression of dental wear, high-concentrated fluoride applications are recommended. Currently, little information is available about the efficacy of other preventive strategies, such as calcium and laser application, as well as the use of matrix metalloproteinase inhibitors.

The term tooth wear is defined as loss of dental hard tissues due to the processes of dental erosion, attrition and abrasion. Dental attrition is the wear of tooth resulting from tooth to tooth contact, while abrasion is caused by oral habits or abrasive substances, such as highly abrasive toothpastes.

At physiological conditions, the oral fluids (saliva, biofilm fluid) have calcium (Ca) and phosphate (Pi) in supersaturated concentrations with respect to the mineral composition of enamel, the outer layer of the tooth, and, as a result, these ions are continually deposited on the enamel surface or are redeposited in enamel areas where they were lost. This can be considered a natural defense phenomenon promoted by saliva to preserve the mineral structure of enamel in the mouth. Therefore, remineralization would be best defined as the redeposition of minerals lost by enamel, and this term has been used as a synonymous of enamel repair or rehardening.

Mineral loss (demineralization) or gain (remineralization) by enamel is a dynamic physicochemical process occurring when oral bacteria form a biofilm on the enamel surface and this biofilm is exposed to fermentable carbohydrates, sucrose being the most cariogenic. Thus, every time sugar penetrates into a cariogenic biofilm and is converted to acids by bacterial metabolism, the biofilm fluid becomes undersaturated with respect to the enamel mineral, and demineralization occurs. A critically low pH for tooth dissolution is maintained for a certain time, but it returns to physiological values when exposure to sugar ceases. Therefore, when the pH is raised and the supersaturating conditions are restored, a certain amount of the mineral lost can be recovered by enamel. This process has been named remineralization. Redeposition of the mineral lost by enamel can occur by Ca and Pi found in the biofilm fluid or by direct action of salivary Ca and Pi soon after the biofilm is removed by toothbrushing. The amount of Ca and Pi gained, however, is lower than that lost, and the net result is a small mineral loss.

If the factors responsible for the disease - biofilm accumulation and frequent sugar exposure - are not controlled, enamel mineral loss cannot be stopped. Repeated events of mineral dissolution will eventually surpass the capacity of oral fluids to repair mineral loss, and the disease will show its first clinical signs: white spot lesions. Differences in the progression rate of caries lesions in different individuals or populations can also be the result of other factors which modulate the caries process, since caries is a "multifactorial" or "complex disease", and the risk of developing new lesions is never zero. In some individuals, lesions will progress slowly, and the disease might not be clinically detected throughout their lifetime. In others, they will progress rapidly, and the manifestation of the disease will become clinically evident (white spots) before cavitation. Additionally, the progression from non-cavitated to cavitated lesions could be arrested, and the strategies used should consider if the lesions are "active or not".

Enamel remineralization has been studied for about 100 years, and it has been suggested that "the non-invasive treatment of early caries lesions by remineralization has the potential to be the major advance in the clinical management of the disease".

One of the key elements of a biological approach in dental treatment is the usage and application of remineralizing agents to tooth structure (enamel and dentin lesions). Known technologies involve casein phosphopeptide stabilized amorphous calcium phosphate (CPP-ACP; RecaldentTM), the second is an unstabilized amorphous calcium phosphate (ACP, EnamelonTM) and the third is a bioactive glass containing calcium sodium phosphosilicate (NovaMinTM). Since all systems rely on calcium and phosphate compounds, their effect is mainly based on an enhancement of the natural capacity of saliva to remineralize mineral loss.

While the incidence of caries has decreased during the past years because of the introduction of water fluoridation and fluoride toothpastes, it is still widespread.

There is a constant need in providing improved therapies, using topical treatments to replace lost calcium and phosphate minerals from early carious lesions that can reduce the need for surgical intervention in the future.

It is the object of the present invention to provide a composition and methods for preventing and treating bone demineralization remineralizing teeth.

The object is achieved by the provision of the embodiments of the present application.

### SUMMARY OF THE INVENTION

The present invention provides a composition comprising calcium carbonate, calcium fluoride and calcium phosphate. Therefore, the composition for oral administration of the present invention can prevent or rapidly treat enamel- and dentin-associated symptoms or diseases, and can provide prolonged prophylactic or therapeutic effects.

Said composition is highly advantageous for remineralizing and desensitizing tooth surfaces, specifically to treat dental erosion or dental caries leading to demineralization of enamel and dentine because said composition can easily be administered and does not have any negative side effects.

Remineralization refers generally to the process of restoring minerals in the form of mineral ions, such as calcium and phosphate ions, to dental enamel and dentine that have been lost due to demineralization.

Yet another object of the invention is to improve remineralizing of children's teeth, while reducing the level of fluoride required for such remineralizing.

Enamel is the outer layer of the tooth and is the hardest substance in the human body which contains the highest percentage of minerals, 96%, with water and organic material composing the rest. The primary mineral is hydroxyapatite, which is a crystalline calcium phosphate. Enamel is formed on the tooth while the tooth is developing within the gum, before it erupts into the mouth. Once fully formed, it does not contain blood vessels or nerves. Remineralization can repair damage to the tooth to a certain degree but damage beyond that cannot be repaired by the body. The maintenance and repair of human tooth enamel is one of the primary concerns of dentistry.

Dentine is a tissue constituting the tooth matrix, and located between dental enamel and dental pulp. Dentin is composed of 70% of inorganic substance, 20% of organic substance and 10% of water. The hardness of dentin is lower than that of dental enamel, but higher than that of cementum. Dentinal tubules throughout the dentin are arranged radially from the surface of the dental pulp towards the dental enamel. The dentinal tubules are wider at the ends near the dental pulp and closer toward the surface. Common dentin-associated diseases or symptoms which cause pain, includes dental caries, tooth wearing, enamel loss and dentin hypersensitivity etc.

According to an embodiment of the invention, the composition comprises calcium carbonate, calcium fluoride and calcium phosphate, wherein calcium carbonate is present in an amount of about 0.25-5g, calcium fluoride is present in an amount of about 0.1-3g, calcium phosphate is present in an amount of about 0.1-3g per unit.

According to a specific embodiment, the composition is free of other minerals or salts, specifically it is free of other calcium salts.

According to a specific embodiment, the composition consists of calcium carbonate, calcium fluoride and calcium phosphate.

More specifically, the composition consists of calcium carbonate, calcium fluoride and calcium phosphate herein calcium carbonate is present in an amount of about 0.25-5g, calcium fluoride is present in an amount of about 0.1-3g, calcium phosphate is present in an amount of about 0.1-3g per unit and optionally further adjuvants and/or preservatives.

According to a specific embodiment, calcium carbonate is present in an amount of between 0.5 and 3g per unit, specifically between 1 and 2.5g, specifically between 1.25 and 2g, more specifically between 1.4 and 1.6g.

According to a specific embodiment, calcium fluoride is present in an amount of between 0.25 and 1.5g per unit, specifically between 0.5 and 1.25g, specifically between 0.5 and 1 g.

According to a specific embodiment, calcium phosphate is present in an amount of between 0.25 and 1.5g per unit, specifically between 0.5 and 1.25g, specifically between 0.5 and 1 g.

According to an embodiment of the invention, the weight ratio of calcium carbonate, calcium fluoride and calcium phosphate is 2.5:1:1.

In a further embodiment, the invention provides a composition comprising calcium carbonate in an amount of about 1.5g, calcium fluoride in an amount of about 0.6g and calcium phosphate in an amount of about 0.6g per unit.

Specifically, the composition consists of 1.5g calcium carbonate, 0.6g calcium fluoride and 0.6g calcium phosphate per unit and optionally further contains adjuvants, additives and/or preservatives.

Adjuvants, additives or preservatives, suspending or scaffolding agents known for production of cosmetic, pharmaceutical or medical compositions are well known in the art and are not defined as active compounds. These may for example be stabilizing agents or adhesion enhancing agents.

According to an embodiment of the invention, calcium carbonate, calcium fluoride and calcium phosphate are homogenously dispersed.

According to a further embodiment, the inventive composition is of immediate release.

In a further embodiment the composition is carrier-free.

Specifically, the composition is a peroral formulation.

The specific dose of compounds administered according to this invention to obtain therapeutic or prophylactic effects can be determined according to the particular circumstances including, for example, the specific route of administration and response of the individual patient, the condition being treated and the severity of the patient's symptoms.

The composition according to the embodiment can be a solid or liquid composition. The composition can be combined into a tablet, gel, hydrogel, polymer, cream, solution, rinse or other pharmaceutically acceptable carrier which can be applied topically to the surface of the teeth.

More specifically a tablet, dispersion, suspension or gel is the most preferred administration form of the inventive composition. Specifically, the composition is formulated to be in contact with the surface of the teeth for prolonged time, specifically it may be a dispersion or gel or tablet that is resolved by saliva and thus provides a dispersion or suspension containing the ingredients of the composition.

According to the terms of the invention, a dispersion is a heterogeneous mixture of calcium carbonate, calcium phosphate and calcium fluoride and optionally further comprising adjuvants, additives or preservatives.

According to the terms of the invention, a suspension is a heterogeneous mixture of calcium carbonate, calcium phosphate and calcium fluoride in a fluid and optionally further comprising adjuvants, additives or preservatives.

More specifically, the composition can be combined with other agents that extend the residence time on tooth surfaces of the respective ingredients calcium fluoride, calcium phosphate and calcium carbonate, while also controlling the rate of release of said remineralizing ingredients onto tooth surfaces.

According to a further embodiment, the composition further comprises flavourings.

A still further object of the invention is to suspense or dispense the remineralizing composition on to tooth surfaces in an aqueous-free emulsion such that, when it comes in contact with saliva, these ingredients form mucoadhesive gels substantive to tooth surfaces; wherein the mucoadhesive gels are subsequently capable of being solubilized by saliva flow, thereby extending the duration of the remineralizing treatment.

In a further embodiment, the inventive composition is formulated as a gel, a paste, a powder, an ointment, a chewing gum, a chewable tablet, a troche, a strip, an emulsion or a glue.

The present invention also provides the inventive composition as a pharmaceutical composition and its use for the preparation of a medicament.

The composition can be specifically used in dental remineralization, specifically for the prevention or treatment of dental caries, dental demineralization, tooth decay, enamel loss, dentin hypersensitivity..

The invention also provides a method for dental remineralization, wherein the composition of the invention is exposed to the teeth for at least 1 minute, preferably at least 2 minutes, preferably at least 3, 4 or 5 minutes, preferably for at least 6, 7, 8, 9 or 10 minutes, specifically for more than 10 minutes.

Specifically, the invention also provides a method for preventing or treating a dentin-associated disease, comprising administering the inventive composition to an oral cavity of a subject.

Thus use of the composition in accord with this invention provides relief to damaged dental tissue, specifically of enamel and dentin.

The examples described herein are illustrative of the present invention and are not intended to be limitations thereon. Different embodiments of the present invention have been described according to the present invention. Many modifications and variations may be made to the techniques described and illustrated herein without departing from the spirit and scope of the invention. Accordingly, it should be understood that the examples are illustrative only and are not limiting upon the scope of the invention.

The invention furthermore comprises the following items:
1. A composition comprising calcium carbonate, calcium fluoride and calcium phosphate, wherein
   calcium carbonate is present in an amount of about 0.25-5g,
   calcium fluoride is present in an amount of about 0.1-3g,
   calcium phosphate is present in an amount of about 0.1-3g per unit.
2. Composition according to item 1, wherein calcium carbonate is present in an amount of about 0.5-3g per unit.
3. Composition according to item 1 or 2, wherein calcium fluoride is present in an amount of about 0.5-1.5g per unit.
4. Composition according to any one of items 1 to 3, wherein calcium phosphate is present in an amount of about 0.5-1.5g per unit.
5. Composition according to any one of items 1 to 4, wherein the weight ratio of calcium carbonate, calcium fluoride and calcium phosphate is 2.5:1:1.
6. Composition according to any one of items 1 to 5 comprising calcium carbonate in an amount of about 1.5g, calcium fluoride in an amount of about 0.6g and calcium phosphate in an amount of about 0.6g per unit.
7. Composition according to any one of items 1 to 6, wherein calcium carbonate, calcium fluoride and calcium phosphate are homogenously dispersed.
8. Composition according to any one of items 1 to 7, which is of immediate release.
9. Composition according to any one of items 1 to 8, which is carrier-free.
10. Composition according to any one of items 1 to 9, which is a solid or liquid composition.
11. Composition according to any one of items 1 to 10, wherein said composition is a peroral formulation.
12. Composition according to any one of items 1 to 11, wherein said composition is formulated as a gel, a paste, a powder, an ointment, a chewing gum, a chewable tablet, a troche, a strip, an emulsion or a glue.
13. Composition according to any one of items 1 to 12 which is a pharmaceutical composition.
14. Composition according to any one of items 1 to 11 for the preparation of a medicament.
15. Composition according to any one of items 1 to 14 for use in dental remineralization.
16. Composition according to any one of items 1 to 14 for use in the prevention or treatment of dental caries, enamel loss, dentin hypersensitivity.
17. A method for dental remineralization, wherein a composition according to any one of items 1 to 13 is exposed to the teeth for at least 1 minute, preferably at least 2 minutes, preferably at least 5 minutes.
18. A method for preventing or treating a dentin-associated disease, comprising administering the composition according to any one of items 1 to 13 to an oral cavity of a subject.

### EXAMPLES

Examples to show efficiency and efficacy of the composition:
Patient, 10 yrs, was diagnosed strong decalcification of the teeth, specifically of the molar teeth. Teeth showed enamel weakness and showed increasing damage. The dentist recommended to extract those teeth (lower molars, sixes). Fluoride compounds were used for years.

Treatment:
Initial motivation and cleaning instruction by dentist, tooth and pocket cleaning by ultrasonic scaling.

Patient was treated with a mixture of 1.5g calcium carbonate, 0.6g calcium fluoride and 0.6g calcium phosphate daily. The composition was contacted with surface of the teeth for about 1 minute and was then swallowed by the patient. The composition was administered for ca. 9 months.

Results were repeatedly controlled by the dentist and it was confirmed that the lesions were significantly reduced.

After 9 months, all teeth, including the molar teeth, looked healthy and fully remineralized. Teeth were looking white, shiny and hard. There was no need to extract the molar teeth (sixes, lower).

## Claims

1. A composition comprising calcium carbonate, calcium fluoride and calcium phosphate, wherein
a. calcium carbonate is present in an amount of about 0.25-5g,
b. calcium fluoride is present in an amount of about 0.1-3g,
c. calcium phosphate is present in an amount of about 0.1-3g per unit.

2. Composition according to claim 1, wherein calcium carbonate is present in an amount of about 0.5-3g per unit.

3. Composition according to claim 1 or 2, wherein calcium fluoride is present in an amount of about 0.5-1.5g per unit.

4. Composition according to any one of claims 1 to 3, wherein calcium phosphate is present in an amount of about 0.5-1.5g per unit.

5. Composition according to any one of claims 1 to 4, wherein the weight ratio of calcium carbonate, calcium fluoride and calcium phosphate is 2.5:1:1.

6. Composition according to any one of claims 1 to 5 comprising calcium carbonate in an amount of about 1.5g, calcium fluoride in an amount of about 0.6g and calcium phosphate in an amount of about 0.6g per unit.

7. Composition according to any one of claims 1 to 6, wherein calcium carbonate, calcium fluoride and calcium phosphate are homogenously dispersed.

8. Composition according to any one of claims 1 to 7, which is of immediate release.

9. Composition according to any one of claims 1 to 8, which is carrier-free.

10. Composition according to any one of claims 1 to 9, wherein said composition is a peroral formulation.

11. Composition according to any one of claims 1 to 10, wherein said composition is formulated as a gel, a paste, a powder, an ointment, a chewing gum, a chewable tablet, a troche, a strip, an emulsion or a glue.

12. Composition according to any one of claims 1 to 11 which is a pharmaceutical composition.

13. Composition according to any one of claims 1 to 10 for the preparation of a medicament.

14. Composition according to any one of claims 1 to 13 for use in dental remineralization.

15. Composition according to any one of claims 1 to 13 for use in the prevention or treatment of dental caries, enamel loss, dentin hypersensitivity.
